# EUROPEAN PATENT APPLICATION

(11) **EP 2 561 810 A1**
(43) Date of publication of application: **27.02.2013**
(21) Application number: 11178730.5
(22) Date of filing: 24.08.2011
(51) Int. Cl.: A61B 5/06, A61B 5/0476, A61B 5/04, G06T 7/00, A61B 5/055

(54) **Method of locating eeg and meg sensors on a head**

(71) Applicant: Université Libre de Bruxelles, 1050 Bruxelles (BE)
(72) Inventor: Engels, Laurent, 1800 Vilvoorde (BE); Warzee, Nadine, 1310 La Hulpe (BE); De Tiege, Xavier, 1460 Virginal-Samme (BE)
(74) Representative: pronovem

(57) **Abstract**

Method of locating EEG and/or MEG sensors provided with reflective markers on a head of a subject, wherein a plurality of cameras are attached to a structure, such that when their positions are fixed relative to the head, and the head is positioned within a target volume in the structure, each marker is seen by at least three cameras. Synchronized two-dimensional images of the reflective markers are captured and three-dimensional coordinates corresponding to each marker are calculated by triangulation. In the same position, part of the face is digitized using a photogrammetry technique involving projection of a light pattern from a fixed location relative to the cameras. The relative position between the digitized face part and the markers' coordinates is resolved. The digitized head surface is matched with the corresponding surface of a 3D MRI image to resolve the locations of the sensors relative to the head.

## Description

The present invention is related to methods and apparatuses for locating electroencephalography (EEG) and/or magnetoencephalography (MEG) sensors on the head of a human or animal subject.

Electroencephalography and magnetoencephalography are non-invasive functional cerebral imaging techniques that record the extracranial electromagnetic fields generated by brain activity. A principal interest of these techniques is to localize within the brain the sources at the origin of the recorded electromagnetic signal. For this purpose, various source localization methods can be used, such as equivalent current dipole or distributed source models.

Sensors used for electroencephalography are electrodes which are placed on the scalp. A number of 19, 32, 64, 128 or even 256 electrodes are typically used, which are distributed over the scalp.

For magnetoencephalography, superconducting quantum interference devices, also referred to as SQUIDs, are used, which are arranged around, but not on the head. The SQUIDs are complemented by head position indicator (HPI) coils, which are placed directly on the scalp. The HPI coils function as sensors which define the position of the head relative to the SQUIDs.

Nowadays, EEG and MEG imaging is often carried out simultaneously. For this purpose, both EEG and MEG sensors often need to be placed together on the scalp.

For an effective EEG/MEG analysis, it is required that the recorded electromagnetic data be referenced in the subject's anatomical space, commonly referred to as co-registration. This is typically performed based on a high-resolution three-dimensional (3D) magnetic resonance imaging (MRI) image of the subject's head. For the analysis, data from at least two, and sometimes three different techniques (EEG, MEG, MRI) hence need to be combined, which requires a precise spatial positioning of the obtained data.

A known way to obtain the spatial positioning of the EEG and MEG sensors relative to a fixed non-anatomical reference is to use an electromagnetic tracking system, such as the Polhemus FASTRAK system (Polhemus, Colchester, USA), which consists of a stylus and an electromagnetic capture device for capturing the spatial position of the stylus. The stylus is manually placed on each sensor and its position is captured. It is evident that such technique is time-consuming, especially when the number of sensors is elevated. Furthermore, for a good accuracy it is required that the subject stands still for the entire measuring period (about one hour), which is difficult to achieve. Additionally, since the stylus will exert a contact force on the sensors, there is an inherent inaccuracy introduced by the compressibility of the skin and any hair present on the scalp. Finally, the measuring space need be free of any magnetic objects in order not to disturb the measurements. These disadvantages are explained more in detail in Engels et al., "Factors influencing the spatial precision of electromagnetic tracking systems used for MEG/EEG source imaging", Clinical Neurophysiology (2010) 40, pp. 19-25.

Another method of computing the spatial position of the sensors is known from US 2005/0058336. Eleven cameras are arranged at the vertices of an icosahedral structure about the head to capture images of the sensors. Each sensor will be seen in at least two images, allowing its position to be established through triangulation. Such a technique permits a faster capture of the sensor locations. However, the structure is geodesic and closely arranged about the head, which may be problematic for persons suffering from claustrophobia.

In order to identify or label the sensors corresponding to the measured positions, the user is required to mark those sensors arranged at the cardinal points of an icosahedron using a graphical user interface. Hence, in the above method, it is required that the sensors be arranged on the head following a well-defined icosahedral pattern, which is furthermore different than the arrangement used traditionally.

The method of US 2005/0058336 hence allows to determine the position of EEG sensors. However, MEG sensors cannot be located with this method, since such MEG sensors are typically placed at random in between EEG sensors.

An additional problem of the above methods, is that they hence allow determining the sensor locations in space, relative to a (fixed) reference, but such reference is not linked to the subject's head or body. Therefore, the obtained sensor locations have to be transformed to the subject's anatomical coordinate system. A known way to do this, is to capture together with the sensor locations, also the locations of a few anatomical landmarks, such as nasion and tragus. Based on the captured anatomical landmark locations, it is possible to position the captured EEG/MEG sensor locations relative to a 3D MRI image of the head. A disadvantage of this procedure is that it can be difficult sometimes to accurately locate the anatomical landmarks, leading to an inaccurate match between EEG/MEG and MRI data.

Another way of co-registration of EEG sensor positioning data with MRI data is known from Koessler et al. in "EEG-MRI Co-registration and Sensor Labeling Using a 3D Laser Scanner", Annals of Biomedical Engineering, Vol. 39, No. 3, pp. 983-995. A precondition set by Koessler in his procedure is that the EEG sensor digitization can be carried out in any medical room and in any light conditions. Therefore, Koessler uses a handheld 3D laser scanner, comprising a laser diode projecting a crosshair on the surface to be digitized and two synchronized cameras which capture an image of the projection. The EEG sensors are attached to a cap, which is placed on the head. Each sensor is provided with a reflective target. In order to be able to calculate the scanner's position in space, at least four reflective target positions need to be identified at all times. Therefore, additional reflective targets are placed on three fiducial landmarks (nasion and two pre-auricular points), two reflective targets are positioned on the cheeks and four on the nose. An operator holding the laser scanner walks around the head scanning it.

Koessler performs a two-step scanning. Firstly, the 3D coordinates of the centre of each reflective target is acquired. Next, the subject's face is scanned with the same device and recorded in the same coordinate system.

Koessler then carries out a co-registration with MRI data based on the acquired face surface only, without taking the sensor positions into account. Koessler reports scanning times of about one minute for the EEG sensors and about two minutes for the face. An accuracy similar to electromagnetic digitizers, but a better repeatability is reported. However, operator skills appear to be important, since Koessler mentions that the scanner should be positioned as perpendicular to the surface to be scanned as possible. Another drawback is that Koessler's method is not completely automatic, since the scans had to be manually inspected to eliminate artefacts, the additional reflective markers on the face and parts of the face surface of no interest.

There is hence a need in the art to provide a method and system of locating EEG/MEG sensors, which overcome the abovementioned drawbacks.

There is a need in the art to provide such methods and systems, which are faster and possibly have an improved overall accuracy, in particular in terms of match between sensor locations and MRI data.

There is also a need in the art to provide such methods and systems, which are more user-friendly, do not require a skilled operator and cause minor disturbance to the subject undergoing analysis.

According to the invention, there is provided a method of locating EEG and/or MEG sensors arranged on a head of a human or animal subject relative to said head, as set out in the appended claims.

In methods according to the invention, each sensor is provided with a reflective marker. A structure is provided, to which a plurality of cameras are attached in a preferably fixed configuration. The subject's head is positioned within a target volume in the structure in a predetermined orientation. The cameras are so attached to the structure, that when their positions are fixed relative to the head, and the head is in the above indicated position and orientation, each marker is seen by at least three cameras. The cameras capture synchronized two-dimensional images of the reflective markers and three-dimensional coordinates corresponding to each marker are calculated, based on triangulation techniques.

According to a first aspect of the invention, for each marker and based on triangulation techniques, three-dimensional coordinates corresponding to the marker are calculated by taking images of the at least three cameras which see the marker into account. The use of at least three images for the calculation of each coordinate results in an improved accuracy and reduces noise effects.

According to a second aspect of the invention, while the subject's head is positioned in the target volume in said predetermined orientation, at least part of the head surface comprising at least part of the face is digitized using a photogrammetry technique involving projection of a light pattern from a fixed location relative to the cameras on the part of the head surface. Next, the relative position between the digitized part of the head surface and the markers' three-dimensional coordinates is resolved. When a three-dimensional magnetic resonance imaging (MRI) image of the subject's head is taken, the digitized head surface can be matched with the corresponding surface of the three-dimensional MRI image to resolve the locations of the sensors relative to the head.

In methods according to the present invention, the above first and second aspects can be implemented separately or in combination.

An advantage of methods of the invention is that, due to the fixed arrangement of both the cameras and the photogrammetry system relative to the head, all images can be taken instantaneously, which reduces inaccuracies due to head movement. Furthermore, no skilled operator is required to acquire the images.

Advantageously, the distance between any one sensor and any one camera during image capture is at least 0.7 m, preferably at least 0.8 m. Such an arrangement optimizes camera incidence angles and avoids claustrophobic effects of the structure. It also allows more freedom of movement to the subject, e.g. to install him/herself in the correct position, without there being a need to have a moving structure as is the case in US 2005/0058336.

Advantageously, methods of the invention comprise generating an envelope of at least part of the head's scalp, based on the calculated marker coordinates. The generated envelope is combined with the digitized part of the head surface to obtain an extended surface. The extended surface is used in matching with the MRI image.

The use of such an extended surface provides increased accuracy when matching with the MRI data.

Advantageously, methods according to the invention comprise capturing multiple sets of synchronized images with the cameras. For each set, the three-dimensional coordinates corresponding to each marker are calculated. Corresponding coordinates between the sets are searched for in order to discard coordinates for which a corresponding coordinate cannot be found in a predetermined number of said multiple sets. This corresponds to the application of a temporal filter on the acquired data in order to stabilize image processing by discarding artefacts, but also when a marker would instantly disappear for an unknown reason. The number of sets is preferably at least three.

The photogrammetry technique advantageously uses a projection of a light pattern, the light of which is not laser light. This will avoid any eye injury to the subject. Hence, methods of the invention are safe, and the subject does not need to keep his/her eyes closed during image acquisition.

Advantageously, digitizing the part of the head surface comprises digitizing the locations of at least some of the sensors and using these digitized locations in resolving the relative position between the digitized part of the head surface and the markers' three-dimensional coordinates.

Advantageously, at least two, preferably between two and six additional reflective markers are placed on the face, preferably on the forehead. In such case, the additional markers are captured in the synchronized two-dimensional images of at least three cameras and their three-dimensional coordinates are calculated. In addition, the locations of the additional markers are digitized together with the part of the head surface with the photogrammetry technique. The calculated coordinates and the digitized locations of the additional markers are then used in resolving the relative position between the digitized part of the head surface and the markers' three-dimensional coordinates.

The additional markers provide a better determination of the relative position between the marker coordinates and the digitized head (face) surface.

Advantageously, the cameras are fixed to a structure having a substantially prismatic shape, wherein the base has a smallest side of at least 1.75 m, preferably at least 2.0 m, and the structure has a height of at least 1.75 m, preferably at least 2.0 m from ground level.

Advantageously, the sensor corresponding to each calculated three-dimensional marker coordinate is identified by manually identifying between one and five, preferably three sensors, followed by fitting a predetermined sensor map to the calculated three-dimensional coordinates. As a result, the manual intervention is kept to a minimum. The few markers that will be identified manually are preferably predetermined markers, advantageously located at easily identifiable positions.

Advantageously, the predetermined sensor map is fitted based on an iterative closest point algorithm.

When both electroencephalography and magnetoencephalography sensors are arranged on the head, the sensor map advantageously contains only data relating to the electroencephalography sensors. The magnetoencephalography sensors are then advantageously identified by three-dimensional coordinates which are outliers (such as coordinates for which no identification could have been calculated) after having fitted the sensor map.

Advantageously, the cameras operate in the infrared and comprise directing infrared light (radiation) on the reflective markers. Illumination of the markers by infrared light originating from the camera locations allows to increase the markers' visibility.

According to another aspect of the invention, there is provided an apparatus for locating EEG and/or MEG sensors arranged on a head of a human or animal subject, relative to said head, as set out in the appended claims. Apparatuses according to the invention comprise at least fourteen first cameras and mounting means for fixedly mounting the first cameras to a structure in such a way that a target volume is defined within the structure, such that when the head is positioned in a predetermined orientation within said target volume and each sensor is provided with a reflective marker, the first cameras are operable to capture images wherein each marker can be seen in the images captured by at least three first cameras. Apparatuses also comprise a device for digitizing a surface, comprising at least a light pattern projector and two spaced apart second cameras fixedly arranged relative to the first cameras, the device being so arranged that it is operable to digitize at least part of the face when the head is positioned in the predetermined orientation within the target volume. In addition, apparatuses comprise a computer and a set of instructions, which when loaded in the computer, are capable of carrying out one or more of the methods according to the invention.
Apparatus of claim 13, wherein and such that.

Advantageously, the mounting means are arranged to attach the cameras to a structure of substantially prismatic shape.

Advantageously, the mounting means are arranged to attach the cameras in such a way to a structure that, when the head is in the predetermined orientation within said target volume, the distance between any one camera and any one sensor is at least 0.7 m, preferably at least 0.8 m.

The structure can be part of apparatuses of the invention. It can as well be a room or other part of a building.

Illustrative embodiments of the present invention will now be described with reference to the attached figures.

Figure 1 represents an EEG electrode placement map for 124 electrodes. Electrodes are named according to the Modified Combinatorial Nomenclature, wherein the 10-20 system electrodes are coloured black.

Figure 2 represents a 17 camera arrangement mounted on a prismatic structure having rectangular base of 3 m by 2.5 m and height of 2.2 m.

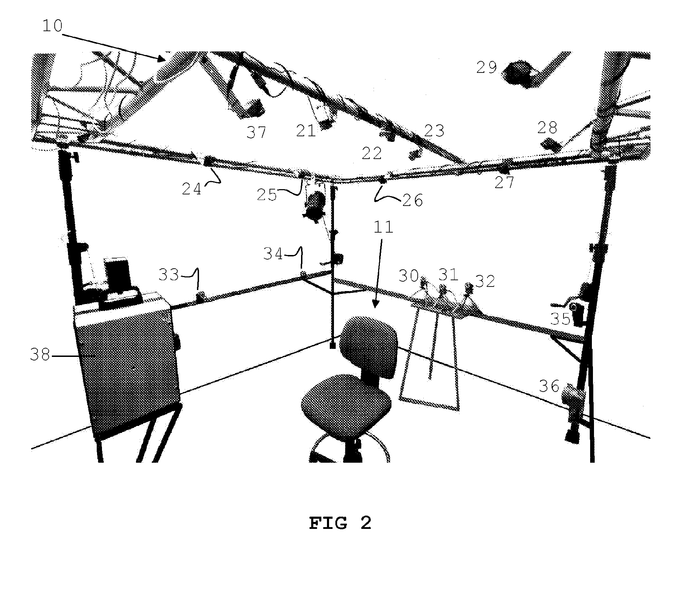

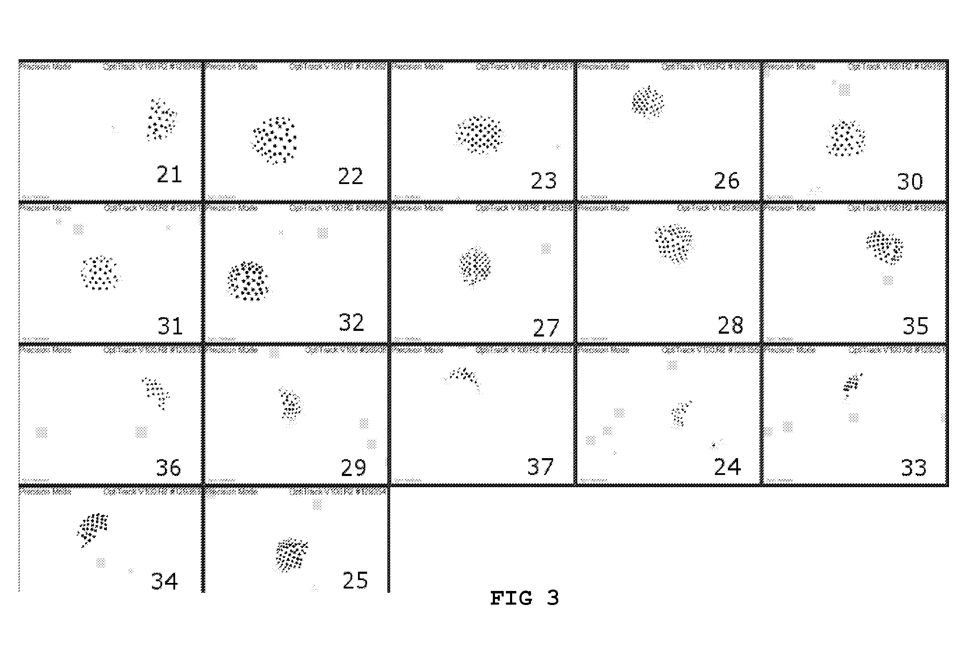

1 Figure 3 represents images acquired with the 17 cameras of figure 2. The numbers in each image correspond to the respective camera of figure 2.

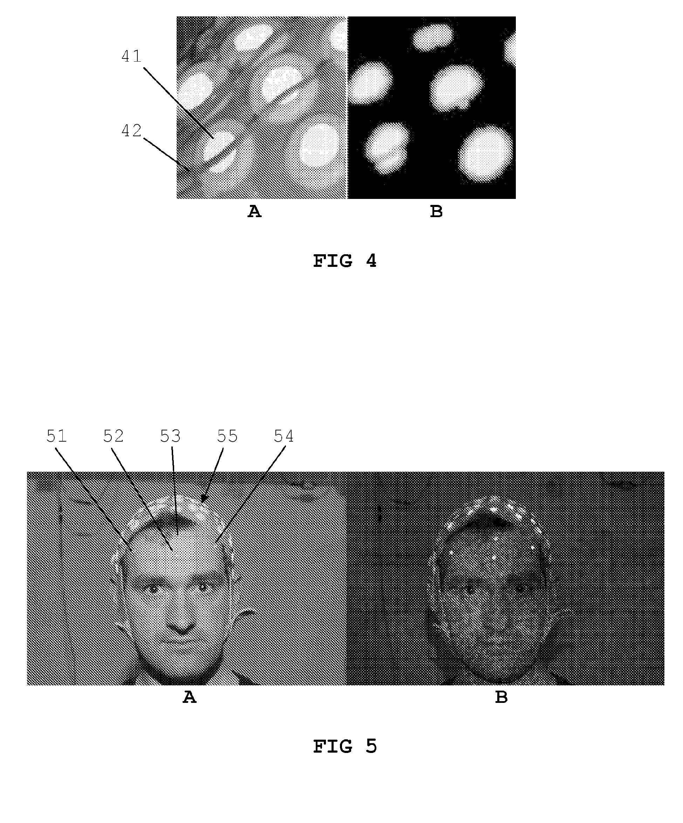

Figure 4 represents reflective markers which are crossed by a wire. Figure 4A is a photograph. Figure 4B is a segmented image.

Figure 5 represents an image of a subject's face. Figure 5A represents a regular photograph, whereas Figure 5B represents an image captured with a photogrammetry camera system when a light pattern is projected onto the face.

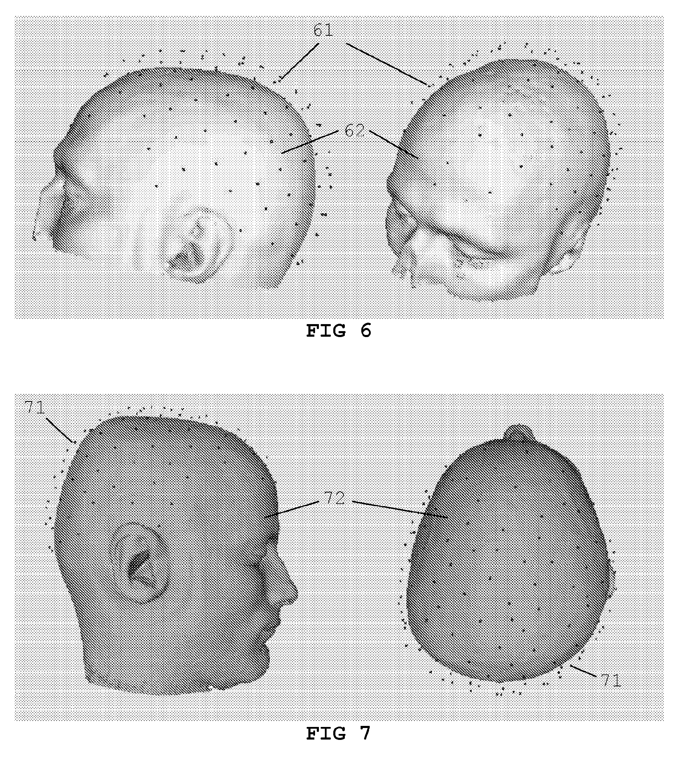

Figures 6 and 7 graphically represent results of locating the marker positions relative to the head of a human subject by matching data calculated with methods of the present invention and MRI data.

Unlike in US 2005/0058336 cited above, the present invention does not require that the EEG electrodes and/or MEG indicator coils be positioned in a well-defined pattern. In fact, methods of the invention will work with any arrangement of such sensors.

The expressions EEG sensor and MEG sensor refer to those elements that are configured to be placed on the head (mainly the scalp) of a subject and which are used to collect data during respectively EEG and MEG tests. EEG sensors are typically electrodes. MEG sensors are the above mentioned HPI coils.

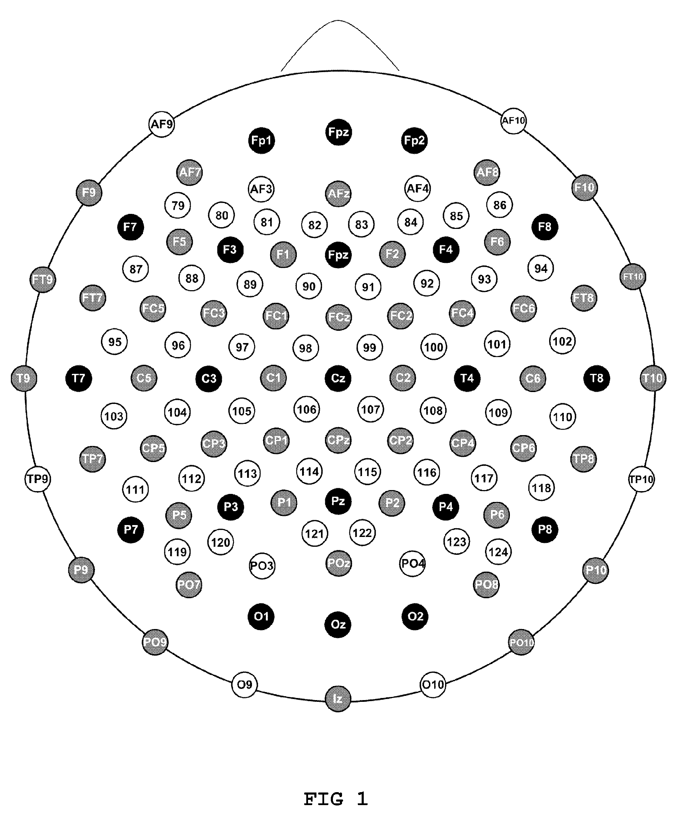

A generally accepted way of EEG sensor placement is according to the internationally recognized 10-20 system. The "10" and "20" refer to the fact that the actual distances between adjacent electrodes are either 10% or 20% of the total front-back or right-left distance of the skull. A total of 19 electrodes can be placed this way, with each electrode labelled with letters F, T, C, P and O according to their position on the scalp (respectively Frontal, Temporal, Central, Parietal, and Occipital). For more detailed EEG analysis, additional electrodes are added utilizing the spaces in-between the existing 10-20 system electrodes. The additional electrodes can be labelled according to the Modified Combinatorial Nomenclature. An extended electrode map is depicted in FIG. 1 for the case of 124 electrodes. The 10-20 system electrodes are coloured black. It is to be noted that other placement systems exist.

The EEG electrodes can be either placed directly on the scalp with an adhesive gel, or attached on an elastic cap, which is then placed over the head. Such a so-called electrocap can be used multiple times.

In case MEG sensors are used, they can be randomly placed in between the EEG sensors, with preferably a minimal distance of 50 mm in between MEG sensors. The number of MEG sensors used is more limited (for example four MEG sensors may suffice). Typically the MEG sensors are so placed that at least one sensor is located in each quadrant (front/back-left/right).

According to the invention, each sensor (EEG and MEG) is provided with a reflective marker, preferably placed on top of it. The reflective marker can be flat and need not be hemispherical. For example, the EEG electrodes can have a 15 mm diameter and a 3.5 mm thickness. They may feature a central hole of 0.5 mm diameter. Reflective markers of 10 mm diameter can be adhered on top of each electrode, preferably at its centre. It is also possible to use markers with a central hole, or markers having same size as the sensors.

Once the sensors with reflective markers are placed at the desired locations on the head, the present invention provides for capturing their locations. This is performed with the aid of a plurality of cameras, which capture two-dimensional images of the markers.

The cameras are preferably infrared imaging cameras, such as the OptiTrack V100:R2 (NaturalPoint, USA). Such a camera has a 640x480 resolution and can capture at a rate of 100 frames per second. In order to reduce the amount of data transfer, the cameras are preferably provided with on-camera image processing capabilities.

It is preferred that light be emitted towards the reflective markers in order to obtain inconspicuous and gentle lighting. Such light is preferably in the infrared, and tuned to the sensitive range of the camera, such as about 850 nm wavelength. The V100:R2 cameras are equipped with a LED ring around the lens emitting 850 nm IR light.

The cameras are fixedly attached to a preferably fixed structure, which in operation surrounds the subject's head. The cameras are so arranged that a target volume is defined within the structure (within the enclosing volume defined by all camera locations), which is preferably at the centre of the structure. In order to minimize the influence of noise during image processing, the arrangement of the cameras is such that when a subject's head is positioned within the target volume, each marker is seen by at least three cameras.

There is no particular constraint on the form of the structure. The structure can hence be dome-shaped. It will however be more convenient to use a prismatic structure, preferably with rectangular base, such as that defined by the walls, floor and ceiling of a room. This avoids the need of providing a special structure for image taking.

The minimal number of cameras depends on the form and dimensions of the structure to which they are attached. For a prismatic structure with rectangular base of 3 m by 2.5 m and height of 2.2 m, a minimum of 12 cameras will be required, but the use of 16 or even 17 cameras will give more accurate results.

Such a structure 10 is shown in FIG. 2. The arrangement of 17 cameras 21-37 is represented as well. The 17 cameras are advantageously attached to the ceiling and walls of the structure, at locations which can be selected in an iterative fashion, without any requirement that they correspond to cardinal points of the structure. In the example of FIG. 2, nine cameras 21-29 are attached to the ceiling, of which three 21-23 directly above the subject's head position. Seven other cameras 30-36 are attached on the walls, slightly below head height (which depends on whether measurements are taken standing or sitting: in case of sitting, the height will be about 1 m), of which three 30-32 at the back wall and two on either side. A 17th camera 37 is attached to the ceiling and directed to the subject's face. Its usage will be explained further below. Reference 11 indicates the position of the head during measurement. The different views of the cameras 21-37 are illustrated in FIG. 3.

As can be seen from FIG. 2, the cameras delimit a volume which is itself substantially prismatic. In the present case it has substantially rectangular base. It will be evident that a base of different shape is possible as well. The base of the volume delimited by the cameras preferably has a smallest internal dimension (from one border to an opposite one) of at least 1.75 m, preferably at least 2.0 m. The height of the delimited volume is preferably at least 1.75 m, preferably at least 2.0 m from ground level.

As an advantage, the minimal distance between cameras and markers is at least 0.7 m, preferably at least 0.8 m. An advantage of the above dimensions is that the structure is less visible to the subject undergoing analysis and is easier to use for the subject and the operator, compared to e.g. the structure represented in US 2005/0058336, which may cause problems to persons suffering from e.g. claustrophobia.

Furthermore, by increasing the distance between cameras and markers/sensors, it is possible to optimize the viewing angle. In addition, the larger distances decrease some of the incidence angles when flat reflective markers are used, such that there is less risk that the reflected light is not seen.

When infrared cameras are used, measurements should preferably be taken in absence of any sunlight, since this would compromise the images. The camera images are therefore preferably captured in the presence of artificial light only.

All cameras are connected to a central computing unit (not shown), which collects the captured and possibly on-camera pre-processed data, for example by USB connections. The central computing can take care of synchronizing the cameras, such that the images are captured in a synchronized way, which avoids any inaccuracy due to head movements.

Once the cameras have been placed, calibration of the system is carried out according to known techniques for triangulation.

As already mentioned, the disposition of the cameras on the structure defines a target volume within which the subject's head should be positioned for meaningful image capturing. The head is positioned within the target volume and in a predetermined orientation in order to capture the images. In FIG 2, the orientation will be such that the head 11 is facing the photogrammetry system 38 (which will be explained further below).

Hence, although the present invention requires a dedicated room for locating the EEG sensors, advantages are that no skilled operator is required to position the subject and acquire the images. Furthermore, since the images are captured synchornized, the data acquisition is immediate, and the subject is not required to stand still for extended time periods. This is particularly advantageous with children.

Once the images of the sensors have been acquired, they are processed in order to extract the sensor locations.

The captured images are advantageously segmented in order to isolate or extract each marker visible on the image. This can be performed on-camera and based on known techniques. Segmentation can be aided by considering the area and/or the aspect ratio of each candidate marker that is isolated. Segmentation will be more robust when reflective markers are used, such that it is based on luminance rather than on colour.

When segmentation is carried out on-camera, it is subsequently transmitted to the computing unit for further analysis, with the goal of calculating 3D coordinates of each marker. Coordinate calculation is based on triangulation techniques, such as described by Hartley and Zisserman in "Multiple View Geometry in computer vision", Cambridge University Press, 2nd edition, 2004. Since each marker is seen by at least three cameras, a three-view geometry approach can be used. For markers that are seen by more than three cameras, the additional images can be taken into account in order to increase accuracy.

A number of filters can be applied to the segmented images, in order to further enhance processing.

For example, a spatial filter can be applied to eliminate false candidate markers, such as in case an electrode wire 42 accidentally passes across a marker 41 as shown in FIG. 4. The spatial filter can unite two candidate markers, when they are too short distanced apart, such as when the distance between their geometrical centres are spaced apart less than a predetermined distance, e.g. 10 mm. This distance will of course depend on the dimensions of the markers used.

Filters for eliminating isolated points can be used.

Elimination of spurious reflections by e.g. the wall or other reflective objects can be accomplished by the application of a window on the image. The window can correspond to a target volume wherein the head is positioned. All identified points which fall outside the window are discarded.

One or more of the above filters can be applied in real-time and can be applied during on-camera processing.

Based on the processed and possibly filtered data, 3D coordinates corresponding to each marker are calculated (established). These coordinates advantageously correspond to the coordinates of the geometrical centres (mass centres) of each marker.

Advantageously, a temporal filter is applied as follows. Synchronized images are captured by the cameras 21-37 at multiple instants of time: t0, t1, t2, etc. Hence, at each time t0, t1, t2 a complete set of synchronized images is acquired. For each set, the 3D marker coordinates are calculated by triangulation. The obtained marker coordinates are then combined or compared between the different sets. For example, a geometrical centre can be calculated between corresponding marker locations of the different sets and its coordinate can be used as a marker coordinate in the further processing.

A particular advantage of the above temporal filter will be apparent in the event that the number of coordinates found differs between the different sets. The decision whether a coordinate is to be retained for further processing or not can be based on the incidence of occurrence of a particular marker location in the different sets. For example, in case three sets of images are acquired and in the event that a found coordinate has a corresponding coordinate in at least one other set, then it can be decided to retain the coordinate for further processing as a marker. If, on the other hand a coordinate only appears in one of the three sets, it can be decided to discard that location. The temporal filter helps in reducing noise and in stabilizing image processing when a marker would instantly disappear for an unknown reason. The temporal filter is advantageously applied to image sets acquired at short intervals. Hence, the time interval t1-t0, t2-t1 between the successive sets is preferably sufficiently short (preferably less than or equal to 100 ms) such that the influence of any head motion can be neglected.

A so called "convex hull" filter can subsequently be applied to eliminate any remaining parasite points. The application of such a filter is described by Dante Treglia in "Game Programming GEMS 3", Charles River Media, 2002, Chapter 3, pp. 273-277. Briefly, a convex hull is the minimal convex set in vector space comprising all calculated points (i.e. corresponding to the calculated coordinates). This can be the minimal convex polyhedron that comprises all the calculated points. After generation of the convex hull, the shortest distance between each point and the closest face of the convex hull is then calculated. When such distance is larger than a predetermined threshold, that point is eliminated.

It is possible to manually eliminate, e.g. through a graphical user interface, any evident parasite points.

The remaining points represent a point cloud of established marker coordinates. They can subsequently be numerically labelled. This label does not correspond to the label of the EEG/MEG sensors. In order to identify the sensor corresponding to each marker, use can be made of a predetermined sensor map, which represents an expected disposition of the sensors on the head. Multiple maps can be registered, and it may be that an operator selects the map that (most closely) corresponds to the actual sensor disposition (e.g. based on the configuration of the electrocap used).

In order to match the sensor map with the point cloud, an operator can be requested, through a user interface, to identify and label a limited number of cloud points, which number can be between one and five, preferably between two and four (e.g. EEG electrodes F_{pz}, C_{z}, and I_{z} as indicated in FIG. 1). Subsequently, the sensor map is fitted to the point cloud, which can involve the application of an iterative closest point (ICP) algorithm, preferably using a non-rigid transformation. More information on the ICP algorithm can be found in "A method for registration of 3-D shapes" by Besl and McKay, IEEE Transactions on Pattern Analysis and Machine Intelligence, 14 (1992), 239-258. Briefly, the ICP algorithm may comprise the following steps. Firstly, points between the two point clouds (the sensor map is also considered a point cloud in this regard) are associated using a nearest neighbour criterion. Secondly, an optimal rigid or non-rigid transformation between the two point clouds is calculated using a least squares approximation. A non-rigid transformation can e.g. be an affine transformation. The transformation is applied to the points of one point cloud and the algorithm is iteratively reapplied until a predetermined error function falls below a predetermined threshold, or until a maximal number of iterations is achieved.

The sensor map need not contain all sensors that are actually placed on the head. For example, the sensor map can relate to EEG sensors only. The MEG sensors can then be found by identifying those points for which the algorithm was unable to find a match. Such points are hence considered outliers to the sensor map. The outliers may be labelled manually.

An automatic control of the number of markers can be carried out based on an operator inputting the total number of sensors for each kind (EEG and MEG).

Compared to the method of US 2005/0058336, methods of the present invention hence allow to further limit the amount of manual intervention. Furthermore, present methods are not restricted to a specific type of sensor arrangement.

The above steps allow establishing the markers' and hence the sensors' locations relative to a machine reference or origin (non-anatomical). Now, the obtained coordinates need to be transformed to an anatomical origin or reference. Only in this way will it be possible to analyse EEG and MEG data accurately.

The problem is tackled starting from the consideration that three-dimensional MRI images give accurate information regarding the subject's anatomy, in particular the head. These three-dimensional MRI images can be obtained by processing two-dimensional MRI images according to known techniques.

In an aspect of the invention, the markers' (or sensors') positions relative to the MRI image are resolved by digitizing part of the head surface, so that the relative position between the digitized surface and the calculated markers' coordinates can be resolved first, and subsequently matching the digitized surface with the corresponding surface of the 3D MRI image.

Hence, instead of locating but a few anatomical landmarks, the entire surface of a part of the head is digitized. Since the upper and rear scalp are covered with EEG/MEG sensors, the surface to digitize will advantageously comprise part of the face, and advantageously corresponds to that part of the face from the upper lip to the forehead, including nasal surface, zygomatic surface, forehead and possibly the eye region. The indicated surface is preferred because it is an anatomically rigid part of the face, which does not change shape between different positions. Conversely, it has been observed that the chin and the throat do change in shape between positions in which MRI measurements are taken and positions in which the EEG/MEG sensors are located. In fact, during MRI, the chin will be more retracted towards the neck, while during EEG/MEG measurements, chin and neck will make an angle of about 90°. Hence, the chin and neck region are advantageously discarded in the above digitization.

It will be advantageous to digitize the selected part of the head with as many points as possible, and hence with a significantly higher point density relative to the sensor density, in order to allow for a good match with the accurate 3D MRI image.

The part of the head surface is preferably digitized with a contactless data acquisition technique, such as a photogrammetry technique, and preferably involves the projection of a light pattern on the surface, such as a structured light. This includes projecting a light pattern on the involved surface and capturing two-dimensional images of the surface with at least two spaced apart cameras.

It will be adavantageous to exploit the structure to which the cameras are mounted and the same positioning of the subject's head. Therefore, the present invention contemplates the use of a fixedly positioned photogrammetry system, advantageously allowing it to cooperate with the fixed camera arrangement as explained below.

The light pattern can be any pattern suitable for this purpose, such as composed of raster lines, different colours, etc. Since the light will mostly be projected in the eye area as well, it is preferred not to use laser light. A system suitable for photogrammetry imaging is a LE-SC 3D camera from Lelee Laser Technology Co. Ltd, China.

The advantage of such a photogrammetry technique is that it is very fast in acquiring images (typically less than a second). Furthermore, it does not interfere with the multiple camera setup for establishing the markers' coordinates. For example, the LE-SC 3D camera works in the infrared as well. Hence, the two kinds of image captures are advantageously carried out simultaneously (they can be synchronized), or at least at close intervals. FIG 2 shows the disposition of the 3D camera 38 relative to the other system elements and the head 11.

A few options are available for resolving the relative positions of the markers relative to the digitized surface. Firstly, the photogrammetry technique can be used to capture the most frontally arranged sensors on the head. Since these sensors have a known geometry and thickness, their location relative to the digitized face surface can be extracted by image analysis (e.g. based on captured texture information) and/or by considering their brightness in the captured images.

Another option contemplated in the present invention is to add a set (e.g. four) of preferably hemispherical reflective markers on the subject's face part which is digitized, in particular on the forehead. These additional markers are captured by the multiple camera system for measuring the markers' locations. To do so, the seventeenth camera 37 can be added to the camera disposition in FIG. 2. This camera is directed to the subject's forehead and frontal part of the scalp.

The additional markers are visible as well in the images captured by the photogrammetry system, as is evident from FIG. 5, showing in FIG. 5B one of two images captured by the above indicated LE-SC camera with projection of structured light, as compared to a normal photograph of FIG. 5A. Note the visibility of the four markers 51-54 placed on the forehead. The electrocap 55 is also visible in FIG. 5A.

Since the position of these four additional markers can be established with both techniques, the digitized surface and the established markers' locations can be brought in correspondence by matching the corresponding locations of the four additional markers.

The above options can be combined such that the relative positions between the digitized surface and the markers' locations can be resolved with higher accuracy. For example, a first attempt to resolve the relative positions of the markers (sensors) relative to the digitized head surface can be made based on the additional markers (preferably between two and six markers), without consideration of any marker placed on the sensors. This model can then be refined/adjusted by considering some of the sensor markers which are also captured by the 3D camera (e.g. using an iterative closest point algorithm). It was found that a combination of four additional markers and four sensor markers give satisfactory results. There is no need to use more sensor markers, since the increase in accuracy will be negligible, yet the computational expense will increase.

With the position of the markers known relative to the digitized head surface, it is now possible to find a match between that model and data from a 3D MRI image in order to locate the sensors relative to an anatomical reference or origin, in particular relative to the brain.

In an aspect of the invention, the digitized part of the head surface (with photogrammetry technique) is matched with a corresponding part of the head surface from the 3D MRI image, possibly based on an iterative closest point algorithm as described above.

The match is advantageously extended by including marker information as follows. An envelope of the scalp is generated based on the marker point cloud. Sensors (markers) which are not arranged on the scalp (e.g. they are arranged too low on the back of the head) may be discarded when generating the envelope. Since the position of the envelope relative to the digitized part of the head (upper face) is known, it can be included in the matching calculations with the MRI data. The scalp envelope can be generated based on a convex hull algorithm as described above. Sensors placed outside the scalp that need to be discarded when generating the scalp envelope can be recognized based on the observation that their surface triangles are much larger than surface triangles between sensor locations placed on the scalp. Possibly, the established marker locations are projected towards the head to compensate for the sensor thickness so that a more accurate envelope can be generated.

By so doing, a very accurate match between MRI data and EEG/MEG sensor data can be obtained.

It should be noted that methods of the invention do not require the presence of both EEG and MEG sensors to be carried out. Methods of the invention can be applied when only EEG sensors are placed on the head. They might as well be applied when only MEG sensors are placed on the head. In case the number of sensors used is limited, a set of additional (dummy) markers may be placed on the head to increase accuracy.

Two tests have been carried out in order to verify the accuracy of the above described methods. In the tests, use has been made of an electrocap comprising 124 EEG electrodes arranged according to the map shown in FIG 1 (15 mm diameter with central hole of 0.5 mm, 3.5 mm thickness) on which flat reflective markers of 10 mm diameter have been placed at their centres. Additionally, four MEG coils were arranged on the electrocap, one in each quadrant and such that MEG coils distanced at least 50 mm from the EEG sensors. The electrocap has been placed on the heads of two human subjects. Four hemispherical markers of 4 mm diameter have been added on the foreheads as shown in FIG 5. The markers' positions were established with the aid of the 17 camera (OptiTrack V100:R2) setup shown in FIG 2 and their faces were digitized using a LE-SC 3D camera as described above and mounted in the position as indicated in FIG 2. Face information below the upper lip was discarded.

The 17 camera images were processed by applying a temporal filter. Three consecutive images were taken with each camera, and objects disappearing in only one of the three images were taken into account, whereas objects disappearing in two of the three images were discarded. Image segmentation was based on area and aspect ratio of the identified marker spots. A spatial filter was applied to unite candidate markers when the distance between their geometrical centres were spaced apart less than 10 mm. Finally, a convex hull algorithm was applied to eliminate false candidates. The remaining candidates were further processed to establish the 3D coordinates of the markers and a point cloud of marker locations was hence obtained.

The digitized upper face data was processed and the four forehead markers were manually extracted. In addition, the four frontmost electrode markers were manually identified as well and their locations established within the digitized surface.

The established marker locations and the digitized upper face were brought in correspondence by matching first the locations of the four forehead markers, and complementing it with the four identified electrode markers using a non-rigid iterative closest point algorithm.

A scalp envelope was generated using the established marker locations. No projection to the head surface was made. A triangle-based surface was generated between marker locations. Marker locations at the corners of surface triangles having an area 4 times larger than the median triangle area were discarded. The scalp envelope was generated by constructing a convex hull with the remaining marker locations.

The scalp envelope was added to the digitized face surface and the combination was matched with a 3D MRI image of each subject using a rigid surface-based iterative closest point algorithm, wherein the MRI data was modified to bring it in correspondence with the digitized/calculated data of face and scalp. FIG 6 and 7 show the obtained results graphically, wherein the marker locations 61 and 71 are represented relative to the heads 62 and 72 (MRI data). For the FIG 6 subject, the mean distance between the electrodes (marker positions) and the MRI model is 4.01 mm ± 2.55 mm. For the FIG 7 subject, the mean distance was 4.38 mm ± 2.42 mm. The distance is of course influenced by any hair present, and includes the thickness of the electrocap and of the electrodes (3.5 mm thickness).

The obtained accuracy is better than the inherent accuracy of the EEG and MEG techniques, so that methods of the invention can be used for reliably localizing the EEG and/or MEG sensors relative to the head.

## Claims

1. Method of locating electroencephalography and/or magnetoencephalography sensors arranged on a head of a subject, relative to said head, comprising:
- providing each sensor with a reflective marker (41),
- providing a structure (10) to which a plurality of cameras (21-37) are attached, such that when the subject's head is positioned in a predetermined orientation within a target volume (11) in the structure and the cameras' positions are fixed relative to the head, each marker is seen by at least three cameras,
- positioning the head in the predetermined orientation in the target volume and capturing synchronized two-dimensional images with the cameras (21-37),
- calculating for each marker, based on triangulation techniques and on captured images of at least three cameras, three-dimensional coordinates corresponding to the marker,
- while the subject's head is positioned in the target volume and in the predetermined orientation, digitizing at least part of the face using a photogrammetry technique involving projection of a light pattern from a fixed location (38) relative to the cameras on the part of the face to obtain a digitized face part,
- resolving the relative position between the digitized face part a n d the markers' three-dimensional coordinates,
- matching the digitized face part with a corresponding surface of a three-dimensional MRI image (62, 72) to resolve the locations of the sensors (61, 71) relative to the head.

2. Method of claim 1, wherein the distance between any one sensor and any one camera (21-37) during image capture is at least 0.7 m, preferably at least 0.8 m.

3. Method of claim 1 or 2, comprising generating an envelope of at least part of the head's scalp, based on the calculated marker coordinates, combining the generated envelope with the digitized face part to obtain an extended surface and using the extended surface in matching with the MRI image (62, 72).

4. Method of any one preceding claim, comprising
- capturing multiple sets of synchronized images with the cameras (21-37),
- for each set calculating the three-dimensional coordinates corresponding to each marker, and
- searching for corresponding coordinates between the sets and discarding a coordinate for which a corresponding coordinate cannot be found in a predetermined number of the multiple sets.

5. Method of any one preceding claim, wherein the photogrammetry technique uses a projection of a non-laser light pattern.

6. Method of any one preceding claim, wherein digitizing the part of the face part comprises digitizing the locations of at least some of the sensors, and wherein the digitized locations are used in resolving the relative position between the digitized face part and the markers' three-dimensional coordinates.

7. Method of any one preceding claim, wherein at least two, preferably between two and six additional reflective markers (51-54) are placed on the face, and comprising:
- capturing the additional markers (51-54) in the synchronized two-dimensional images of at least three cameras (21-37),
- calculating the three-dimensional coordinates thereof,
- digitizing the locations of the additional markers (51-54) together with the part of the face with the photogrammetry technique, and
- using the calculated coordinates and the digitized locations of the additional markers in resolving the relative position between the digitized face part and the markers' three-dimensional coordinates.

8. Method of claim 7, wherein the additional reflective markers (51-54) are placed on the forehead.

9. Method of any one preceding claim, wherein the cameras (21-37) are fixed to a structure (10) having a substantially prismatic shape, wherein the base has a smallest side of at least 1.75 m, preferably at least 2.0 m, and the structure has a height of at least 1.75 m, preferably at least 2.0 m from ground level.

10. Method of any one preceding claim, comprising identifying the sensor corresponding to each calculated three-dimensional marker coordinate, by manually identifying between one and five, preferably three sensors followed by fitting a predetermined sensor map to the calculated three-dimensional coordinates.

11. Method of claim 10, wherein the predetermined sensor map is fitted based on an iterative closest point algorithm.

12. Method of claim 10 or 11, wherein both electroencephalography and magnetoencephalography sensors are arranged on the head, wherein the sensor map contains only data relating to the electroencephalography sensors and wherein the magnetoencephalography sensors are identified by three-dimensional coordinates which are outliers after having fitted the sensor map.

13. Method of any one preceding claim, wherein the cameras (21-37) operate in the infrared and comprising directing infrared radiation on the reflective markers.

14. Apparatus for locating electroencephalography and/or magnetoencephalography sensors arranged on a head of a subject, relative to said head, comprising:
- at least fourteen first cameras (21-37) and mounting means for fixedly mounting the first cameras (21-37) to a structure (10) in such a way that a target volume (11) is defined within the structure (10), such that when the head is positioned in a predetermined orientation within said target volume and each sensor is provided with a reflective marker (41), the first cameras (21-37) are operable to capture images wherein each marker (41) can be seen in the images captured by at least three first cameras,
- a device (38) for digitizing a surface, comprising at least a light pattern projector and two spaced apart second cameras fixedly arranged relative to the first cameras, the device (38) being so arranged that the device is operable to digitize at least part of the face when the head is positioned in the predetermined orientation within the target volume,
- a computer and a set of instructions, which when loaded in the computer, are capable of carrying out the method of any one preceding claim.

15. Apparatus of claim 14, wherein the mounting means are arranged to attach the cameras to a structure of substantially prismatic shape and such that when the head is in the predetermined orientation within said target volume, the distance between any one camera and any one sensor is at least 0.75 m.
